# EUROPÄISCHE PATENTANMELDUNG

(11) **EP 3 421 990 A1**
(43) Veröffentlichungstag der Anmeldung: **02.01.2019**
(21) Anmeldenummer: 17001111.8
(22) Anmeldetag: 29.06.2017
(51) Int. Cl.: G01N 33/483, G01N 33/493, G01N 22/00

(54) **EXKREMENTEANALYSE MITTELS RADARWELLEN**

(71) Anmelder: Herbst, Martin, 5090 Lofer (AT)
(72) Erfinder: Herbst, Martin, 5090 Lofer (AT)

(57) **Zusammenfassung**

Die Erfindung besteht aus einem Gerät, mit dem Körperausscheidungen (fest oder flüssig) analysiert werden können, ohne dass die Sensoren mit dem zu analysierenden Material in Kontakt kommen.

## Beschreibung

Die vorliegende Erfindung betrifft ein Gerät, mit dem Körperausscheidungen (fest oder flüssig) drahtlos analysiert werden können. Dies geschieht in einem Orthogonal-Frequenzverfahren (Radar) in Frequenzbändern von 433 MHz, 800 MHz, 2,5 GHz, 3,5 GHz, 5 GHz, 10 GHz, 60 GHz und 210 GHz. Die Erfassung und Analyse erfolgt, ohne dass die Sensoren mit dem zu analysierenden Material in Kontakt kommen.

Zum Erfassen und Separieren der Proben können auch Kapillarröhrchen eingebaut werden, mit einem Durchmesser von wenigen Milimetern bis ein Tausendstel Milimeter, damit eine noch genauere Trennung und Analysierung der Proben möglich ist.

Die gewonnen Messdaten werden automationsunterstützt verarbeitet. Die Messsensoren sind imstande, über mehrere hundert Messergebnisse zu liefern. Diese Messwerte werden an ein anderes Gerät (PC bzw. Rechner, sonstiges mobiles Gerät) gesendet und werden dort ausgewertet. Die ermittelten Daten geben dann Rückschlüsse über den augenblicklichen körperlichen Zustand desjenigen, dessen Probe analysiert wurde. Der PC bzw. Rechner ermittelt Empfehlungen für den idealen körperlichen Zustand, bezogen auf vorzuwählende Daten wie Alter, Gewicht, medizinischer Zustand des Probanden und aller anderer möglichen Einflüsse. Um in diesen gewünschten Idealzustand zu gelangen, wählt der PC aus einer Datenbank Vorschläge, insbesondere hinsichtlich Ernährung, Getränke und Speisen aus und in weiterer Folge auch die Art und Weise der Bewegung, des herbeizuführenden Kalorienverbrauchs und zukünftigen Lebensart (Sport, Bewegung, Schlaf, Belastung usw.). Nach Einnahme der vorgeschlagenen Stoffe, Getränke oder Speisen, sowie der vorgeschlagenen Betätigung bzw. Bewegung des Körpers, wird der optimale Wohlfühlzustand und somit die Normalwerte für den jeweiligen Körper erreicht. Nachfolgend ergibt eine Berechnung die zukünftige Leistungsfähigkeit in Bezug auf Kraft, Ausdauer in physischer und psychischer Hinsicht und eine Berechnung der Lebenserwartung unter Anführung aller lebensnahen Parameter incl. Umwelteinflüsse und Belastung des Körpers. Mit diesem Analysegerät können auch jegliche, insbesondere kritische Gesundheitszustände angezeigt werden.

Bisher ist es äußerst umständlich, aktuelle Messwerte über den Körperzustand zu bekommen, insbesondere über die Ausscheidungsprodukte des menschlichen Körpers, den Urin und den Kot. Hiezu bietet sich derzeit die Möglichkeiten der Auswertung bei einem Hausarzt, der die Urin- bzw. Kotprobe in ein Labor schicken muss und dann die Messwerte ausgewertet zurückerhält oder es sind Streifen in einer Apotheke erhältlich, die über einen bestimmten Messwert des menschlichen Körpers Auskunft geben können. Sind die Messwerte bekannt, erhält man in der Praxis oft nur unzureichend Information darüber, welche Getränke und Nahrungsmittel eingenommen werden sollen, um damit den gewünschten Erfolg zu erhalten. Hausärzte sind keine Ernährungswissenschafter. Hinzu kommt, um das Ergebnis und die aktuellen Messwerte zu kontrollieren, dass ein erneuter Arztbesuch bzw. ein neuer Streifen nötig ist. Die anderen Grundelemente (WC, Pissoir, automationsunterstützte EDV) mit diesen Messwerten, Daten bzw. Informationen vereint, hat es bis dato in dieser Form nicht gegeben. Insbesondere wird der Effekt der Selbstheilung, im Körper bestens unterstützt, um auch problematische Körper in den ursprünglichen optimalen Zustand zurückzuversetzen.

Ziel der Erfindung ist eine Herstellung eines Systems, das den Menschen in die Lage versetzt, schnell und ohne großen Aufwand bei Benützung des WC bzw. Pissoirs Informationen über seinen Körper und zugleich eine Empfehlung über jene Lebensmittel und Bewegungsabläufe zu erhalten, die bei Einnahme bzw. Durchführung eine entsprechende positive Wirkung und lebensverlängernde Wirkung ausüben und die Messwerte in den gewünschten optimalen Zustand (Wohlfühlzustand) bringt. Die nachgeschaltete Rechnereinheit nimmt Auswertungen aufgrund ernährungswissenschaftlichermedizinischer- und sportwissenschaftlicher Kenntnisse sowie aller jeweils verfügbarer Messwerte vor. So können Auswertungen generiert werden, wie z.B. Abweichungen von Normalwerten, aber es kann auch in weiterer Folge ein Menü erstellt werden, um die gemessenen Körperwerte in den Normalzustand, bzw. in den gewünschten Köperzustand (Wohlfühlzustand) hinüberzuführen.

## Patentansprüche

1. Ein Gerät, mit dem Körperflüssigkeiten sowie Körperausscheidungen (fest oder flüssig) drahtlos analysiert werden können. Die Analyse geschieht in einem Orthogonal- und anderen Frequenzverfahren (Radar) in Frequenzbändern von 433 MHz, 800 MHz, 2,5 GHz, 3,5 GHz, 5 GHz, 10 GHz, 60 GHz und 210 GHz und über 300 GHz.

2. Ein Gerät nach Anspruch 1, wobei zur Trennung und Erfassung der Proben Kapillarröhrchen mit einem Durchmesser von wenigen Milimetern bis ein Tausendstel Milimeter verwendet werden.
